Europäisches Patentamt

European Patent Office

Office européen des brevets

(11) Publication number: **0 111 875**
**B1**

## EUROPEAN PATENT SPECIFICATION

(45) Date of publication of patent specification: **23.07.86**

(51) Int. Cl.⁴: **A 61 M 11/02, B 05 B 11/04**

(21) Application number: **83112522.4**

(22) Date of filing: **13.12.83**

(54) Metered dose spray dispenser.

<table>
<tr><td>

(30) Priority: **20.12.82 US 451311**

(43) Date of publication of application:
**27.06.84 Bulletin 84/26**

(45) Publication of the grant of the patent:
**23.07.86 Bulletin 86/30**

(84) Designated Contracting States:
**AT BE CH DE FR GB IT LI NL SE**

(56) References cited:
**FR-A-2 312 421**
**US-A-4 168 032**

</td><td>

(73) Proprietor: **SCHERING CORPORATION**
**2000 Galloping Hill Road**
**Kenilworth, New Jersey 07033 (US)**

(72) Inventor: **Hegemann, Manfred Karl**
**Ferris Lane**
**South Nyack New York 10960 (US)**
Inventor: **Drozd, Edward Joseph, Jr.**
**10 Maureen Court**
**Lake Hiawath New Jersey 07034 (US)**

(74) Representative: **von Kreisler, Alek et al**
**Patentanwälte Von Kreisler-Schönwald-Fues-**
**Keller Selting-Werner Deichmannhaus am**
**Hauptbahnhof**
**D-5000 Köln 1 (DE)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates generally to spray dispensers and, more specifically, to an improved metered dose spray dispenser for pharmaceuticals, especially to one that can be held in the hand.

The dispensing of pharmaceuticals into a body cavity, for example a nostril, has generally required a squeeze bottle having a nozzle which is inserted into the cavity. The amount of liquid dispensed with each squeeze of the bottle usually depends on how the person squeezes it. Thus, one cannot be sure of reproducibly accurate delivery. Since dosage consistency is important in treating a medical condition, a pump which meters the drug amount is needed. Typical examples of prior art metering pumps are shown in U.S. Patents 2,434,875, 3,820,698 and 3,949,939, and in United Kingdom Patent Specification 1,517,642.

In each of the above devices, which are merely examples, the metering pump reciprocates along an axis coincident with the nozzle dispensing axis to pump metered portions of liquid from the container through the nozzle. The forces applied by the users are along the axis of pumping and dispensing. The user grasps the dispenser between his thumb and fingers and squeezes. Generally what happens is that the nozzle is removed from the target area during dispensing by the force and action required to operate the pump. This is both inconvenient and undesirable since the product does not reach the intended site and often splashes the face.

Other types of atomizers or manual metered spray pumps have applied the force along the axis of reciprocation of the pump and dispense liquid along an axis transverse to the pumping actions. These generally include a deflector or other device at the output of the pump to redirect the liquid. Typical examples are shown in U.S. Patents 3,900,138, 4,082,222, 4,088,425 and 4,089,442. Even though these patents specifically show the axis upon which the manual force is applied as being traverse to the axis of dispensing, the force is applied along the axis of the pump.

Another alternative is to use a trigger mechanism which receives a manual force transverse to the pumping axis and thus to dispense the fluid through a nozzle which is transverse to the dispensing axis. This is illustrated in U.S. Patent 4,077,548.

The trigger and an adapter are positioned on the top of the dispensing bottle and manually actuate the pump by pivoting about a hinge point. This U.S. patent shows that the lateral component of the saddle member on the pushbutton of the pump is thereby minimized. Although this trigger mechanism may be an improvement over the prior art, it is clumsy and is generally not acceptable for use in small dose applicators of pharmaceuticals, for example, nasal sprays. Since the trigger extends substantially away from the body of the device, the dispenser can be accidentally actuated if carried in the user's purse or pocket. Thus, this configuration is not practical for a pharmaceutical which must be available to the users several times a day.

An object of the present invention is to provide an improved hand-held manual meter pump.

The invention therefore provides a metered-dose spray dispenser suitable for manual actuation and comprising:

a housing

a nozzle extending from said housing and shaped for insertion into a body orifice for directing a spray along a dispensing axis;

a container movably mounted inside said housing for holding liquid to be dispensed;

manual pump means interconnecting said nozzle and said container for pumping a metered amount of liquid from said container to said nozzle in a single pumping cycle;

orifice means in said housing for providing access to said container from outside said housing along a manual force axis transverse to said dispensing axis; and

converting means pivotally connected to said housing and engaging said container for converting a manual force applied through said orifice means along said manual force axis into a force moving said container along said pumping axis to activate said pump means and dispense a metered amount of liquid through said nozzle.

The converting means can include a first portion substantially following the contour of said housing across said orifice means, and a second portion inside said housing substantially transverse to said first portion and engaging said container whereby manual force applied to said first portion through said orifice means along said manual force axis moves said container along said pumping axis by virtue of said second portion.

In one embodiment, the converting means is a hinge pivotally mounting the container in the housing with a portion of the container adjacent the orifice whereby the manual force is applied directly to the container. The surface of the container juxtaposed the orifice in the housing has a high coefficient of friction, to prevent slipping of the fingers upon application of the pumping force. The housing, nozzle and a first hinge portion are a unitary molded structure. The container may include an integrally formed second hinge portion to cooperate with the first hinge portion to connect the container pivotally to the housing; alternatively a separate hinge portion may be used and secured to the container by the pump structure.

In another embodiment, the converting means is an L-shaped cross-section structure pivotally connected to the housing with a first portion substantially following the contour of the housing across the orifice and a second portion within the housing substantially orthogonal to the first portion and engaging the container. The converting means and housing may be a unitary molded structure or separate structures.

For the better understanding of the invention, particularly preferred embodiments thereof will be described with reference to the accompanying drawings, wherein

Figure 1 is a perspective view of a metered spray dispenser with the cap open incorporating the principles of the present invention;

Figure 2 is a side elevation of the metered spray dispenser of Figure 1 with the cap closed and with the side wall cut away to show the liquid container and pump in its initial position;

Figure 3 is the metered spray dispenser of Figure 2 with the container and pump in its final position after dispensing:

Figure 4 is a side elevation of a container with a separate and distinct hinge member;

Figure 5 is a perspective view of the hinge member of Figure 4;

Figure 6 is a side elevation of another embodiment of a metered spray dispenser with the cap off incorporating the principles of the present invention;

Figure 7 is a back elevation of the embodiment of Figure 6 with the cap on the housing; and

Figure 8 is a side elevation of the embodiment of Figure 6 with the cap on and the side wall and cap cut away.

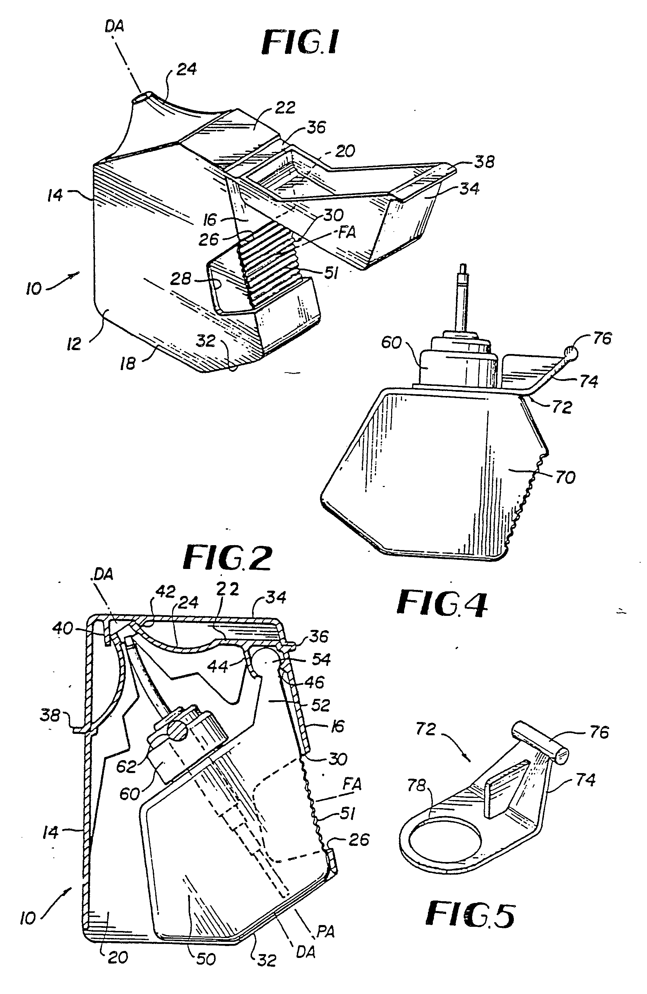

A hand held metered spray dispenser 10 includes a housing 12, a container 50 for liquid, and a manually actuated pump to dispense liquid from the container 50 through a nozzle 24 on the housing 12. As specifically illustrated in Figure 1, housing 12 includes a front wall 14, a back wall 16 and side walls 18 and 20. A top wall 22 includes a nozzle 24 having a dispensing axis DA. The nozzle 24 is tapered and has a generally conical shape for ease of insertion into a nostril or other body cavity. The back wall 16 includes an orifice 26, and the side walls 18 and 20 include indentures 28 and 30 respectively which are contiguous with the orifice 26, thereby forming substantially a slot.

Container 50 is substantially the shape of the housing 12 and has its bottom edge matching the bottom of the housing 12. A portion 51 of the rear wall of the container 50 juxtaposed the orifice 26 in the housing is shown as a plurality of grooves. The orifice 26 has an axis FA through which a manual force can be applied to the container 50 in a direction which is substantially transverse to the dispensing axis DA. These grooves on portion 51 of container 50 form a highly frictional surface to prevent slipping of the finger which will apply the force directly to container 50 through the orifice 26 and indentures 28 and 30 of the housing 12. The bottom of the housing 12 is open at 32 to provide for loading and unloading the container.

A cap 34 is connected to the top wall 22 by a living hinge 36. The cap 34 includes a rim 38 which extends past the front wall 14 of the closed position as illustrated in Figure 2 to aid in lifting the cap 34 to open the dispenser 10. As illustrated specifically in Figure 2, attached to the inside of the cap 34 are a pair of spaced longitudinal ribs 40 and 42 which engage the nozzle 24 when the cap 34 is in the closed position and act as a lock mechanism for the cap 34, thereby preventing evaporation and spillage. Also illustrated in Figure 2 is a lip 44 extending down from the interior of the top wall 22 and a lip 46 on the back wall 16 to define a confined space there between. The lips 44 and 46 form a mounting element of a hinge for the container 50 within the housing which can rotate therein, whereby manual force along axis FA is converted into a force along the dispensing axis DA (as will be described more fully below). An extension 52 of the container terminates in a cylindrical section 54 which is received between the lips 44 and 46 of the housing 12 to form the second portion of a hinge for the container 50 and to mount the container 50 pivotally inside the housing 12. It is preferred that the container 50, the extension 52 and hinge element 54 are formed as a unitary molded structure.

As is evident from the drawing, the dispensing axis DA of the nozzle 24 is inclined relative to the front wall 14. The side wall 16 is substantially parallel to the dispensing axis DA. This specific configuration allows the nozzle 24 to be inserted within a nostril while still leaving room for the thumb to engage the front wall 14.

Preferably the housing 12, the nozzle 24, the cap 34, the living hinge 36 and the lips (mounting element) 44 and 46 are a unitary molded structure. Although this is an optimum cost-effective way of manufacturing, it should be noted that these pieces may be separate and distinct for assembly into the complete dispenser. It should also be noted that although the dispenser is illustrated as being a box-shape, the front, back and sides may be a continuous surface in some continuous curve. However, it is important that the orifice 26 with the indentures 28 and 30 allows entry of a finger on grooved portion 51 of container 50 to a substantial depth to the interior of the housing 12 when manual force is applied along axis FA for dispensing the contents of the container 50.

Connecting the interior of the container 50 to the nozzle 24 is a pump 60. The pump 60 includes a spring 62 which returns the compressed pump components to their extended position when the pumping force is removed. These pumps also generally include check valves, and accumulators or reservoirs such that a metered amount of liquid is drawn into the pump reservoir while the pump extends and this metered amount is dispensed while the pump contracts. The specific details of such pumps are well known and do not need to be discussed. Any suitable pumping structure, e.g. one disclosed in the prior art mentioned herein, may be used.

The spring 62 of the pump is used to maintain the container 50 in its initial position as illustrated in Figure 2 and to return it from its pivoted position of Figure 3 back to the position of Figure 2 after the manual force is removed. As illustrated in Figure 2, the pumping axis PA is substantially coincident with the dispensing axis DA. It is evident from Figure 3 that the pumping axis PA of the pump will be coincident with the dispensing axis DA in the contracted position. As is well known, the meter pump 60 contracts and expands reciprocally along the pumping axis PA to dispense a metered amount of liquid as a spray.

In use, the cap 34 is removed exposing the nozzle 24 which is inserted into a body orifice. The housing is grasped in the hand with one finger on the grooved portion 51 of the container in the orifice 26 with the remainder of the fingers wrapped around the housing. Upon application of the force directly onto the container 50 along the force axis FA transverse to the dispensing axis DA, the container rotates from the initial position of Figure 2 to the position in Figure 3. This rotation causes the portions of the pump to contact the interior of the housing and contract. This contraction dispenses a metered portion of liquid as a spray from the container 50 through the nozzle 24, with the pumping axis PA aligned with the dispensing axis DA. Upon release of the manual force, the spring 62 of the pump returns the container 50 to its initial position illustrated in Figure 2.

The dispenser 10 can be used to dispense drugs, in particular expensive life-saving drugs, for example interferon, into the nostrils. Because of the expense, the pump must be very accurate and capable of dispensing small amounts each time. The liquid in container 50 may also be a colloid solution. The pump should be capable of dispensing metered amounts in the range of 0.02 to 0.2 milliliters and preferably in the range of 0.04 to 0.1 milliliters. The pump should have a dosage reproducibility in the range of 50% to 100% and preferably in the range of 80% to 100%.

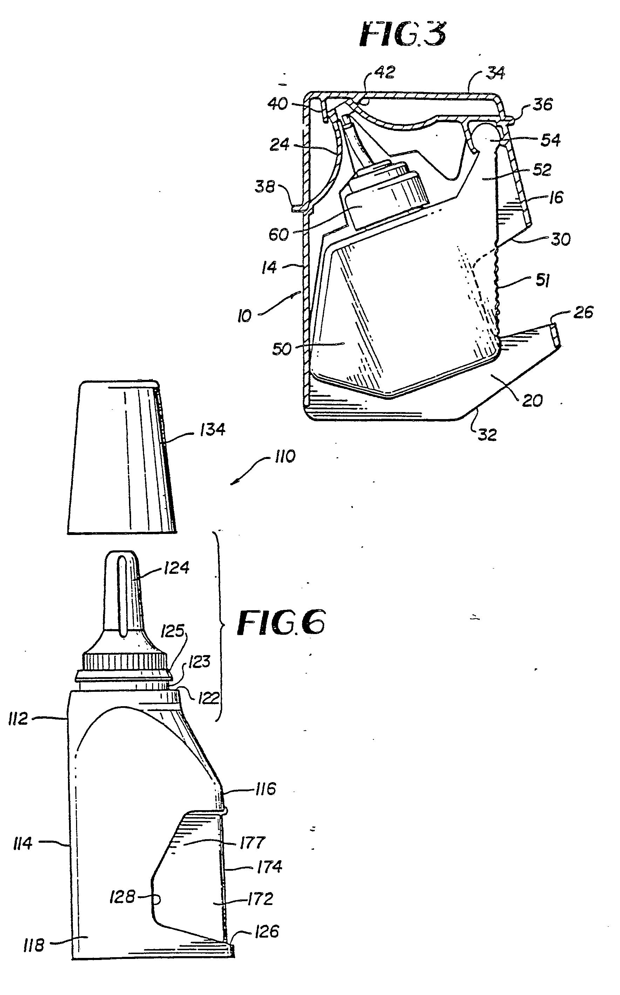

As with the housing, the preferred embodiment for the container 50, the extension 52 and the hinge 54 is a unitary molded structure. Alternatively, as illustrated in Figures 4 and 5, the container 70 may be distinct and separate from the hinge element 72. The shape of the container 70 is substantially that of container 50, illustrated in Figure 2, except that the extension 52 and the hinge element 54 are absent. Instead a hinge element 72 includes an extended portion 74 terminating in a cylindrical hinge 76 at one end and an orifice 78 within the hinge member 72 at the other end. This orifice 78 is received on the neck of the container 70 and is held thereto by the pump 60 which is generally screwed onto the top of the container 70. Alternatively, a hinge element can be atttached to the bottom or other area of the container. The embodiment illustrated in Figure 4 functions just like the container 50 illustrated in Figures 2 and 3.

Another embodiment of the present invention is illustrated in Figures 6, 7 and 8. For ease of understanding, the embodiment of Figures 6, 7 and 8 will have 100 added to the numbers of the embodiment of Figures 1 through 5. The metering dispenser 110 includes a housing having front wall 114, back wall 116, and side walls 118 and 120. As top wall 122 has a collar 123 therein through which extends a nozzle 124. The back wall 116 includes an orifice 126 and the side walls 118 and 120 include indentures 128 and 130 respectively contiguous with the orifice 126. The orifice 126 and the indentures 128 and 130 substantially form a slot. A cap 134 engages and is held on the top wall 122 by a rim 125 on the collar 123.

The nozzle 124 is part of a base 127. The interior of the housing 110 includes an extended portion 129 which engages the circumference of the base 127 of the nozzle 124 and maintains the nozzle mounted to the housing. A container 170 has external threads 171 which are mounted to a pump means similar to that of Figures 1 through 5 which is not shown for sake of clarity in Figures 6 through 8. The pump means is provided between the container 170 and the nozzle 124 with the base 127 forming part of the pump. The container 170 moves along the coincident pumping and dispensing axis PA and DA.

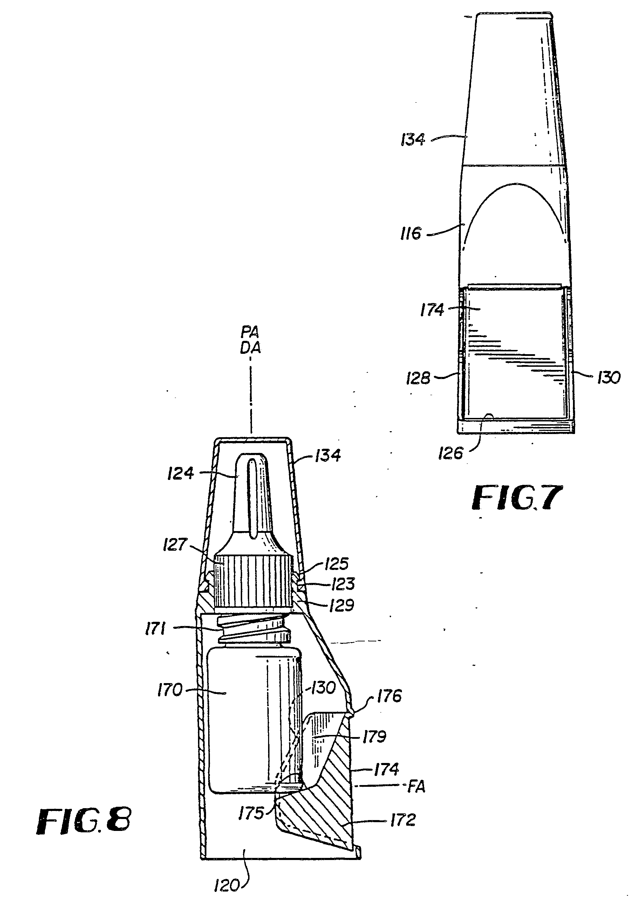

The converter or the element which converts the manual force applied along force axis FA through the orifice 126 to move the container 170 along the pumping axis PA is shown in Figures 6 through 8 as a hinged element 172 having a generally L-shaped cross-section with portions 174 and 175 illustrated in Figure 8. Portion 174 extends across the orifice 126 and follows the contour of the back wall 116. The portion 175 which is substantially orthogonal to the portion 174 of the converted hinge 172 provides a camming surface which engages the bottom of the container 170. The converter 172 is hinged at 176 to the back wall housing 116 by, for example, a living hinge. Thus, converter 172 is a unitary molded structure with the housing 112. Lateral walls 177 and 179 provide an enclosure around the sides of the container 170 and fill the void of indentures 128 and 130 of the housing.

Although the bottom edge of the converter 172 extends past the orifice 126 and engages the interior of back wall 116 to maintain the converter 172 within the housing, it should be noted that portion 174 may extend exterior the housing back wall 116 without departing from the present invention. The important thing is that it does not extend substantially beyond the housing in order to prevent accidental operation of the device during transit. It should also be noted that although pivot 176 is shown as a living hinge and an integral part of the converter 172 and housing 112, the converter 172 may be a separate and distinct element and may be pivotally connected at any suitable place within the interior of the housing 112. Similarly, the converter may be hinged at the corner of the L-shape instead of at the ends of one of the legs. It should also be noted that although housing 112 is illustrated as a closed molded device, it may also be formed as a unitary molded device with a living hinge along one of the walls such that it may be opened up to allow insertion of the container 170 other than along the pumping and dispensing axis PA and DA.

The embodiment of Figures 6 and 8 operates substantially as does that of Figures 1 through 5; a manually applied force along force axis FA onto portion 174 of the converter 172 causes pivotal movement of the converter 172 about pivotal axis 176 so that surface 175 forces the container 170 along the pumping axis PA, thereby actuating the pump to dispense liquid from the container along dispensing axis DA and out through nozzle 124. The user holds the housing 112 in the hand and needs only a single finger to actuate converter 172. As in the previous embodiment, this minimizes the amount of motion of the housing 112 and of the nozzle 124 thereby providing accurate placement of the dispensed liquid.

It is evident from this detailed description of these embodiments of the invention that a new and improved metered dose spray dispenser is provided. This dispenser overcomes the problems of the prior art by applying the manual activation force of the metered pump on an axis transverse to the dispensing axis and transverse to the pumping axis, so minimising lateral displacement of the nozzle. Since most of the hand encompasses the housing versus the container and the nozzle is integral to the housing, lateral motion is minimized. In prior art devices, the hand engages a substantial portion of the container and applies the force in a substantially different direction relative to the dispensing axis and the pumping axis.

**Claims**

1. A metered dose spray dispenser suitable for manual actuation and comprising:
   a housing (12, 112);
   a nozzle (24, 124) extending from said housing and shaped for insertion into a body orifice for directing a spray along a dispensing axis (DA);
   a container (50, 70, 170) movably mounted inside said housing for holding liquid to be dispensed;
   manual pump means (60) interconnecting said nozzle and said container for pumping a metered amount of liquid from said container to said nozzle in a single pumping cycle and acting along a pumping axis (PA);
   orifice means (26, 126) in said housing for providing access to said container from outside said housing along a manual force axis (FA) transverse to said dispensing axis; and
   converting means (52, 72, 172) pivotally connected to said housing and engaging said container for converting a manual force applied through said orifice means along said manual force axis into a force moving said container along said pumping axis to activate said pump means and dispense a metered amount of liquid through said nozzle.

2. A metered spray dispenser as claimed in claim 1 wherein said pumping axis (PA) is substantially coincident with said dispensing axis (DA) and wherein said pump means preferably includes return means (62) for returning said pump means and said container to their initial operating position.

3. A metered spray dispenser as claimed in claim 1 or claim 2, wherein said converting means (52, 72, 172) either includes hinge means for pivotally mounting said container to the interior of said housing with a side of said container adjacent to said orifice means whereby said manual force is applied to said container through said orifice means, wherein said pivotal mounting hinge means includes a first portion (44, 46) unitarily molded with said housing (12) and a second portion (52, 72) unitarily molded with said container or secured to said container by said pump means; or includes a first portion (174) substantially following the contour of said housing (112) across said orifice means, and a second portion (175) inside said housing substantially transverse to said first portion and engaging said container whereby manual force applied to said first portion through said orifice means (126) along said manual force axis moves said container along said pumping axis by virtue of said second portion, especially wherein said orifice means includes a first portion whose axis is parallel to said manual force axis and opposed second and third portions (128, 130) whose axes are transverse to said manual force axis to form a slot in said housing means.

4. A metered spray dispenser as claimed in any of claims 1 to 3 wherein the surface (51) of said container (50, 70) juxtaposed said orifice means (26) has a high coefficient of friction, preferably by having a plurality of grooves.

5. A metered spray dispenser as claimed in any of claims 1 to 4 wherein said housing includes a cap means (34) hinged at one side to said housing means for covering said nozzle means during non-use, preferably wherein said housing means, said nozzle means and said cap means are a unitary molded structure.

6. A metered spray dispenser as claimed in any of claims 1 to 5 wherein said housing (12) includes a top wall (22) and four side walls (14, 16, 18, 20), said nozzle (24), preferably shaped for insertion into a nostril, being in said top wall, said orifice means (26) being in one (16) of said side walls, and wherein two of said side walls (18, 20) each adjacent to an opposite edge of said one side wall each preferably include an indenture (28) contiguous with said orifice in said one side wall to form a slot therewith, in particular wherein said one side wall (16) is substantially parallel to said nozzle axis and a side wall opposite said one side wall is inclined relative to said dispensing axis.

7. A metered spray dispenser as claimed in any of claims 1 to 6 wherein said converting means (172), is pivotally connected to said housing (112) and includes a first portion (174) across said orifice means and a second portion (175) inside said housing substantially transverse to said first portion and engaging said container for converting a manual force applied to said first portion through said orifice means along said manual force axis into a force moving said container by said second portion along said pumping axis and activating said pump means to dispense a metered amount of fluid through said nozzle, in particular wherein said converting means is substantially L-shaped in cross-section and is pivotally connected to said housing at the end (176) of said first portion not common to said second portion, or wherein said first portion of said converting means substantially follows the contour of said housing across said orifice means.

8. A metered spray dispenser as claimed in any of claims 1 to 7 wherein said container contains a colloid medical solution.

9. A metered spray dispenser as claimed in any of claims 1 to 8 wherein said pumping means pumps in the range of 0.02 to 0.20 milliliters, preferably 0.04 to 0.10 milliliters, or liquid in a single pumping cycle.

10. A metered spray dispenser as claimed in any of claims 1 to 9 wherein said pumping means pumps a metered amount of liquid having a reproducibility in the range of 50% to 100%, preferably in the range of 80% to 100%

**Patentansprüche**

1. Handbetätigbarer Zerstäuber mit dosierter Abgabe, bestehend aus

einem Gehäuse (12, 112);

einer Düse (24, 124), die von dem Gahäuse ausgeht und zur Einführung in eine Körperöffnung geformt ist, um einen Spühnebel längs einer Ausgabe-Achse (DA) zu richten;

einem in dem Gehäuse beweglich angeordneten Behälter (50, 70, 170) zur Aufnahme von abzugebender Flüssigerkeit;

einer Handpumpvorrichtung (60), die die Düse und den Behälter verbindet und dazu dient, in einem einzigen Pumpzyklus eine dosierte Flüssigkeitsmenge aus dem Behälter zu der Düse zu pumpen und längs einer Pumpachse (PA) zu wirken;

einer Öffnungsanordnung (26, 126) in dem Gehäuse, die von außerhalb des Gehäuses längs einer zu der Ausgabe-Achse quer verlaufenden Handkraftachse (FA) Zugang zu dem Behälter verschafft und

einer Umwandlungsvorrichtung (52, 72, 172), die drehbar mit dem Gehäuse verbunden ist und an den Behälter angreift, um eine durch die Öffnungsanordnung längs der Handkraftachse ausgeübte Handkraft umzuwandeln in eine Kraft, die den Behälter zur Betätigung der Pumpvorrichtung und zur Abgabe einer dosierten Flüssigkeitsmenge durch die Düse längs der Pumpachse bewegt.

2. Dosier-Zerstäuber nach Anspruch 1, bei dem die Pumpachse (PA) im wesentlichen mit der Ausgabe-Achse (DA) zusammenfällt und bei dem die Pumpvorrichtung vorzugsweise eine Rückstelleinrichtung (62) zur Rückführung der Pumpvorrichtung und des Behälters in ihre Ausgangs-Arbeitsposition aufweist.

3. Dosier-Zerstäuber nach Anspruch 1 oder 2, bei dem die Umwandlungsvorrichtung (52, 72, 172) entweder eine Gelenkanordnung zur drehbaren Lagerung des Behälters im Inneren des Gehäuses aufweist, wobei eine Seite des Behälters bei der Öffnungsanordnung liegt, so daß die Handkraft durch die Öffnungsanordnung auf den Behälter ausgeübt wird, bei dem die Drehlager-Gelenkanordnung einen mit dem Gehäuse (12) einheitlich geformten ersten Teil (44, 46) und einen mit dem Behälter einheitlich geformten bzw. an dem Behälter mit Hilfe der Pumpvorrichtung befestigten zweiten Teil (52, 72) aufweist; oder einen der Kontur des Gehäuses (112) über die Öffnungsanordnung hinweg im wesentlichen folgenden ersten Abschnitt (174) und einen zweiten Abschnitt (175) innerhalb des Gehäuses aufweist, der zu dem ersten Abschnitt im wesentlichen quer verläuft und an den Behälter angreift, so daß durch die Öffnungsanordnung (126) längs der Handkraftachse auf den ersten Abschnitt ausgeübte Handkraft den Behälter mit Hilfe des zweiten Abschnittes längs der Pumpachse bewegt, insbesondere bei dem die Öffnungsanordnung einen ersten Teil mit zu der Handkraftachse paralleler Achse sowie gegenüberliegende zweite und dritte Teile (128, 130) aufweist, deren Achsen zu der Handkraftachse quer verlaufen, um einen Schlitz in der Gehäuseanordnung zu bilden.

4. Dosier-Zerstäuber nach einem der Ansprüche 1 bis 3, bei dem die neben der Öffnungsanordnung (26) liegende Fläche (51) des Behälters (50, 70) einen hohen Reibungskoeffizienten aufweist und vorzugsweise mit mehreren Rillen versehen ist.

5. Dosier-zerstäuber nach einem der Ansprüche 1 bis 4, bei dem das Gehäuse eine Kappenanordnung (34) aufweist, die an einer Seite an die Gehäuseanordnung angelenkt ist, um die Düsenvorrichtung bei Nichtbenutzung abzudecken, vorzugsweise bei dem die Gehäuseanordnung, die Düsenanordnung und die Kappenanordnung einen einheitlichen Formkörper bilden.

6. Dosier-Zerstäuber nach einem der Ansprüche 1 bis 5, bei dem das Gehäuse (12) aus einer Oberwand (22) und vier Seitenwänden (14, 16, 18, 20) besteht, die vorzugsweise zur Einführung in ein Nasenloch geformte Düse (24) in der Oberwand angeordnet ist und die Öffnungsanordnung (26) sich in einer (16) der Seitenwände befindet und bei dem zwei der Seitenwände (18, 20) jeweils bei einem gegenüberliegenden Rand der einen Seitenwand jeweils vorzugsweise einen Ausschnitt (28) aufweisen, der an die Öffnung in der einen Seitenwand angrenzt, um mit dieser einen Schlitz zu bilden, insbesondere bei dem die eine Seitenwand (16) im wesentlichen parallel zu der Düsenachse verläuft und eine der einen Seitenwand gegenüberliegende Seitenwand (14) in bezug auf die Ausgabeachse geneigt ist.

7. Dosier-Zerstäuber nach einem der Ansprüche 1 bis 6, bei dem die Umwandlungsvorrichtung (172) schwenkbar mit dem Gehäuse (112) verbunden ist und einen sich über die Öffnungsanordnung hinweg erstreckenden ersten Abschnitt (174) sowie einen zweiten Abschnitt (175) innerhalb des Gehäuses aufweist, der im wesentlichen quer zu dem ersten Abschnitt gerichtet ist und an den Behälter angreift, um eine durch die Öffnungsanordnung längs der Handkraftachse auf den ersten Abschnitt ausgeübt Handkraft umzuwandeln in eine Kraft, die den Behälter vermittels des zweiten Abschnittes längs der Pumpachse bewegt und die Pumpvorrichtung zur Abgabe einer dosierten Fluidmenge durch die Düse betätigt, insbesondere bei dem die Umwandlungsvorrichtung im wesentlichen L-förmigen Querschnitt aufweist und an dem Ende (176) des ersten Abschnittes, das nicht zu dem zweiten Abschnitt gehört, drehbar mit dem Gehäuse verbunden ist oder bei dem der erste Abschnitt der Umwandlungsvorrichtung in vesentlichen der Kontur des Gehäuses über die Öffnungsanordnung hinweg folgt.

8. Dosier-Zerstäuber nach einem der Ansprüche 1 bis 7, bei dem der Behälter eine medizinische Kolloidlösung enthält.

9. Dosier-Zerstäuber nach einem der Ansprüche 1 bis 8, bei dem die Pumpvorrichtung im Bereich von 0,02 bis 0,02 Milliliter, vorzugsweise 0,04 bis 0,10 Milliliter der Flüssigkeit in einem einzigen Pumpzyklus pumpt.

10. Dosier-Zerstäuber nach einem der Ansprüche 1 bis 9, bei dem die Pumpvorrichtung eine dosierte Flüssigkeitsmenge mit einer Reproduzierbarkeit im Bereich von 50% bis 100%, vorzugsweise im Bereich von 80% bis 100% pumpt.

**Revendications**

1. Pulvérisateur à dosage approprié à l'actionnement manuel et comprenant:

un boîtier (12, 112);

un gicleur (24, 124) s'étendant à partir dudit boîtier et conformé en vue de son insertion dans une cavité du corps humain afin de diriger un jet pulvérisé le long d'un axe d'éjection (DA);

un récipient (50, 70, 170) monté mobile à l'intérieur dudit boîtier pour contenir un liquide à pulvériser;

un moyen formant pompemanuelle (60) reliant entre eux ledit gicleur et ledit récipient pour pomper une quantité dosée de liquide dudit récipient vers ledit gicleur en un seul cycle de pompage et agissant le long d'un axe de pompage (PA);

un moyen formant orifice (26, 126) dans ledit boîtier pour permettre l'accès audit récipient de l'extérieur dudit boîtier le long d'un axe de force manuelle (FA) transversal audit axe d'éjection; et

un moyen convertisseur (52, 72, 172) monté pivotant sur ledit boîtier et en contact avec ledit récipient pour convertir une force manuelle appliquée à travers ledit moyen formant orifice le long dudit axe de force manuelle en une force déplaçant ledit récipient le long dudit axe de pompage afin d'actionner ledit moyen de pompage et d'éjecter une quantité dosée de liquide à travers ledit gicleur.

2. Pulvérisateur à dosage comme revendiqué dans la revendication 1, dans lequel ledit axe de pompage (PA) coïncide sensiblement avec ledit axe d'ejection (DA) et dans lequel ledit moyen formant pompe comprend de préférence un moyen de retour (62) pour ramener ledit moyen formant pompe et ledit récipient à leur position de fonctionnement initial.

3. Pulvérisateur à dosage comme revendiqué dans la revendication 1 ou la revendication 2, dans lequel ledit moyen convertisseur (52, 72, 172) comprend soit un moyen d'articulation pour le montage pivotant dudit récipient sur l'intérieur dudit boîtier de telle manière qu'un côté dudit récipient soit adjacent audit moyen formant orifice de sorte que ladite force manuelle soit appliquée audit récipient à travers ledit moyen formant orifice; dans lequel ledit moyen d'articulation pour montage pivotant comprend une première partie (44, 46) moulée d'une seule pièce avec ledit boîtier (12) et une deuxième partie (52, 72) moulée d'une seule pièce avec ledit récipient ou rendue solidaire dudit récipient par ledit moyen formant pompe; soit comprend une première partie (174) suivant sensiblement le contour dudit boîtier (112) d'un côté à l'autre dudit moyen formant orifice, et une deuxième partie (175) à l'intérieur dudit boîtier sensiblement transversale à ladite première partie et en contact avec ledit récipient de sorte qu'une force manuelle appliquée à ladite première partie à travers ledit moyen formant orifice (126) le long dudit axe de force manuelle déplace ledit récipient le long dudit axe de pompage grâce à ladite deuxième partie; dans lequel, en particulier, ledit moyen formant orifice comprend une première partie dont l'axe est parallèle audit axe de force manuelle et des deuxième et troisième parties opposées (128, 130) dont les axes sont transversaux audit axe de force mauelle de maniére à former une rainure dans ledit moyen formant boîtier.

4. Pulvérisateur à dosage comme revendiqué dans l'une quelconque des revendications 1 à 3, dans lequel la surface (51) dudit récipient (50, 70) juxtaposée audit moyen formant orifice (26) présente un haut coefficient de frottement, en étant de préférence pourvue d'une pluralité de cannelures.

5. Pulvérisateur à dosage comme revendiqué dans l'une quelconque des revendications 1 à 4, dans lequel ledit boîtier comporte un moyen formant couvercle (34) articulé d'un côté audit moyen boîtier pour recouvrir ledit moyen formant gicleur pendant la non utilisation; de préférence dans lequel ledit moyen formant boîtier, ledit moyen formant gicleur et ledit moyen formant couvercle constituent une structure moulée d'une seule pièce.

6. Pulvérisateur à dosage comme revendiqué dans l'une quelconque des revendications 1 à 5, dans lequel ledit boîtier (12) comprend une paroi supérieure (22) et quatre parois latérales (14, 16, 18, 20), ledit gicleur (24), de préférence conformé en vue de son insertion dans une narine, se situant dans ladite paroi supérieure, ledit moyen formant orifice (26) se situant dans une première (16) desdites parois latérales, et dans lequel deux desdites parois latérales (18, 20), adjacentes chacune à un bord opposé de ladite première paroi latérale comportent de préférence chacune un enfoncement (28) contigu audit orifice de ladite première paroi latérale de manière à former avec lui une gorge; dans lequel, en particulier, ladite première paroi latérale (16) est sensiblement parallèle audit axe du gicleur et une paroi latérale (14) opposée à ladite première paroi latérale est inclinée par rapport audit axe d'éjection.

7. Pulvérisateur à dosage comme revendiqué dans l'une quelconque des revendications 1 à 6, dans lequel ledit moyen convertisseur (172) est monté pivotant sur ledit boîtier (112) et comprend une première partie (1974) s'étendant d'un côté à l'autre dudit moyen formant orifice et une deuxième partie (175) située à l'intérieur dudit boîtier, sensiblement transversale à ladite première partie et en contact avec ledit récipient afin de convertir une force manuelle appliquée à ladite première partie à travers ledit moyen formant orifice le long dudit axe de force manuelle en une force déplaçant ledit récipient par ladite deuxième partie le long dudit axe de pompage et activant ledit moyen formant pompe pour éjecter une quantité dosée de fluide à travers ledit gicleur; dans lequel, en particulier, ledit moyen convertisseur est sensiblement en forme de L en section transversale et est monté pivotant sur ledit boîtier à l'extrémité (176) de ladite première partie qui n'est pas commune à ladite deuxième partie, ou dans lequel ladite première partie dudit moyen convertisseur suit sensiblement le contour dudit boîtier d'un côte à l'autre dudit moyen formant orifice.

8. Pulvérisateur à dosage comme revendiqué dans l'une quelconque des revendications 1 à 7, dans lequel ledit récipient contient une solution médicamenteuse colloîdale.

9. Pulvérisateur à dosage comme revendiqué dans l'une quelconque des revendications 1 à 8, dans lequel ledit moyen de pompage effectue le pompage de 0,02 à 0,20 millilitres, de préférence 0,04 a 0,10 millilitres, de liquide en un seul cycle de pompage.

10. Pulvérisateur à dosage comme revendiqué dans l'une quelconque des revendications 1 à 9, dans lequel ledit moyen de pompage effectue le pompage d'une quantité dosée de liquide dont la reproductibilité est comprise dans la plage de 50% à 100%, de préférence dans la plage de 80% à 100%.

# FIG.1

# FIG.2

# FIG.4

# FIG.5

FIG.3

FIG.6

FIG.7

FIG.8